# FASCICULE DE BREVET EUROPEEN

(11) **EP 0 737 181 B1**
(45) Date de publication et mention de la délivrance du brevet: **24.02.1999**
(21) Numéro de dépôt: 95903847.2
(22) Date de dépôt: 16.12.1994
(51) Int. Cl.: C07C 209/48, C07C 211/12

(54) **PROCEDE D'HYDROGENATION CATALYTIQUE DE NITRILES EN AMINES EN PRESENCE D'UN CATALYSEUR DE TYPE NICKEL DE RANEY DOPE**
VERFAHREN ZUR KATALYTISCHER HYDROGENIERUNG VON NITRILEN ZU AMINEN IN ANWESENHEIT EINES DOSIERTEN RANEY NICKEL KATALYSATORS
METHOD FOR THE CATALYTIC HYDROGENATION OF NITRILES INTO AMINES IN THE PRESENCE OF A DOPED RANEY NICKEL TYPE CATALYST

(30) Priorité: 28.12.1993 FR 9316008
(43) Date de publication de la demande: 16.10.1996
(73) Titulaire: RHODIA FIBER & RESIN INTERMEDIATES, 92408 Courbevoie Cédex (FR)
(72) Inventeur: CORDIER, Georges, F-69340 Francheville (FR); FOUILLOUX, Pierre, F-69300 Caluire-et-Cuire (FR); LAURAIN, Nathalie, F-69003 Lyon (FR); SPINDLER, Jean-Francis, F-69003 Lyon (FR)
(74) Mandataire: Vignally, Noel
(86) Numéro de dépôt international: FR9401478
(87) Numéro de publication internationale: WO9518090

(56) Documents cités:
- US-A- 3 821 305
- US-A- 3 862 911
- US-A- 4 248 799
- RUSSIAN CHEMICAL REVIEWS, vol.33, no.6, Juin 1964, MOSCOW, RU pages 319 - 330 L. K. FREIDLIN ET. AL. 'Catalytic Reduction of Dinitriles' cité dans la demande

## Description

La présente invention se rapporte au domaine très vaste de la réduction catalytique de nitriles, en particulier de mononitriles et/ou dinitriles, en monoamines, diamines ou aminonitriles par mise en oeuvre de catalyseurs de Raney dopés par un ou plusieurs éléments métalliques d'addition.

Plus précisément, la présente invention a pour objet un procédé d'hydrogénation de nitriles en amines, par exemple de mononitriles et/ou dinitriles, en monoamines et/ou diamines, à l'aide d'un catalyseur du type nickel de Raney dopé par au moins un élément métallique d'addition choisi dans le groupe IVb de la classification périodique et issu d'un alliage précurseur métallurgique Ni/Al/élément(s) dopant(s).

La réduction de nitriles en amines, par exemple de dinitriles en diamines, est une transformation chimique qui revêt la plus grande importance en chimie industrielle, car les amines, et en particulier les diamines, sont des composés très utilisés comme monomères réactifs dans des réactions de polycondensation, par exemple avec d'autres monomères bifonctionnels. Un exemple particulièrement illustratif de cette dimension industrielle à grande échelle est celui de l'adiponitrile susceptible d'être hydrogéné en hexaméthylènediamine. Ce dernier composé est l'un des monomères de base de fabrication du polyamide-6,6, dont on connaît l'importance économique.

Cette réduction de nitriles en amines doit, également, s'entendre comme comprenant la transformation de dinitriles en aminonitriles, par exemple d'adiponitrile en aminocapronitrile, ce dernier pouvant être converti en caprolactame par hydrolyse cyclisante. Le caprolactame est, lui aussi, un composé fondamental en chimie des fibres polymères, puisqu'il constitue le monomère du polyamide-6.

De façon traditionnelle, l'hydrogénation des nitriles en amines s'opère avec le soutien catalytique de nickel de Raney, éventuellement dopé. Ces catalyseurs sont préparés par lixiviation de l'aluminium d'alliages AI-Ni riches en aluminium, en milieu fortement alcalin. Les catalyseurs obtenus sont constitués par des agglomérats de cristallites de nickel, ayant une importante surface spécifique et une teneur résiduelle en aluminium variable.

La modification des facteurs structuraux et électroniques du nickel de Raney, par l'addition de métaux à l'alliage nickel-aluminium, a déjà été envisagée. Classiquement, l'addition d'un dopant s'effectue par introduction dans un alliage précurseur Ni-AI en fusion. Il s'agit du dopage métallurgique. Ainsi, le dopage de nickel de Raney par divers promoteurs métalliques (Fe, Co, Cr, Mn, V, Mo, Zr, Ta, Ti), ainsi que leurs incidences quant à l'activité, la sélectivité et la stabilité du catalyseur, font l'objet d'une riche littérature scientifique et technique.

L'article de FREIDLIN et al *(Russian Chemical Review,* vol. 33, n°6, june 1964) traite de la réduction catalytique de dinitiles et fait l'inventaire d'un certain nombre de catalyseurs de Raney dopés, mis en oeuvre dans des conditions d'hydrogénation (température, pression en hydrogène (PH2), milieu réactionnel) variées. Des nickels de Raney dopés au chrome, au cuivre et au titane sont notamment cités. Avec le Ni de Raney dopé au chrome, l'hydrogénation s'opère dans l'anhydride acétique en présence de NaOH à une température de 50°C et une pression en hydrogène d'environ 0,35 MPa, pour obtenir une sélectivité de 77 % en diamine à partir d'adiponitrile. En ce qui conceme le Ni de Raney dopé au titane, le milieu réactionnel contient du butanol et de l'ammoniaque, la température est de 140°C à 180°C, la pression d'hydrogène de 14 MPa environ et la sélectivité en diamine est de 60 % à partir de dicyanobenzène. Il apparaît également que les éléments dopants (tels que Ti) sont présents dans une quantité supérieure ou égale à 4 % en poids par rapport au nickel.

On constate que les sélectivités obtenues dans les hydrogénations de dinitriles à l'aide de Ni de Raney dopé, décrites dans ce document antérieur, sont relativement faibles. En outre, dans certains cas, les conditions réactionnelles, telles que la température et la pression d'hydrogène, sont à des niveaux si élevés qu'ils nuisent à la commodité de mise en oeuvre du procédé, ainsi qu'à son économie.

Le brevet **FR-A 2 068 953** conceme des Ni de Raney dopés au chrome par voie métallurgique.

Un premier inconvénient de l'emploi du chrome comme dopant tient au fait que ce métal est susceptible dans certains cas d'être considéré comme pouvant poser des problèmes en matière de pollution.

Un deuxième inconvénient est que le chrome ne permet pas d'atteindre des teneurs négligeables en impuretés d'hydrogénation de nitriles comme par exemple le diaminocyclohexane (DCH). Or, ces impuretés sont particulièrement gênantes, car elles ont sensiblement la même température d'ébullition que les amines visées et sont donc très difficiles à éliminer.

Il existe donc un besoin industriel certain d'optimisation des conditions d'hydrogénation de nitriles en amines, notamment des. dinitriles en aminonitriles et/ou diamines, au moyen de catalyseurs de type Ni de Raney dopés, notamment au regard des conditions opératoires, ainsi que de l'activité, la sélectivité et la stabilité du catalyseur final.

Une telle optimisation constitue l'un des objets essentiels de la présente invention, qui comprend un procédé d'hydrogénation de nitriles en amines facile à mettre en oeuvre, non polluant, économique et permettant, d'une part, d'atteindre des sélectivités en diamine supérieures à 90 % exprimées par rapport au substrat nitrile de départ et, d'autre part, de réduire au maximum les impuretés.

Il s'agit donc plus précisément d'un procédé d'hydrogénation de nitriles en amines à l'aide d'un catalyseur du type Ni de Raney, caractérisé en ce que :
- ledit catalyseur est dopé par au moins un élément métallique d'addition choisi dans le groupe IVb de la classification périodique et issu d'un alliage précurseur métallurgique Ni/Al/élément(s) dopant(s),
et en ce que ledit procédé consiste essentiellement:
- à sélectionner un milieu réactionnel liquide et solvant du substrat nitrile à hydrogéner,
- à mettre en oeuvre au moins une base minérale choisie parmi les hydroxydes de métal alcalin ou alcalinoterreux
- et à adopter un catalyseur dont le rapport pondéral élément dopant/Ni, est compris entre 0,05 et 10 %.

Il est du mérite de la Demanderesse d'avoir mis au point un procédé d'hydrogénation performant résultant d'un compromis technique entre, d'une part, un catalyseur Ni de Raney dopé avec des quantités déterminées d'éléments d'addition et, d'autre part, des conditions réactionnelles choisies avec soin.

Ainsi, le catalyseur Ni de Raney dopé mis en oeuvre dans ce procédé provient d'un alliage précurseur Ni-AI (teneur en Ni de 28 à 59 % en poids par poids), mis en fusion et additionné d'au moins un élément métallique d'addition, de préférence le titane, selon une procédure de dopage dite "métallurgique". Après refroidissement, l'alliage précurseur dopé est soumis, de manière traditionnelle, à une attaque alcaline provoquant une élimination plus ou moins importante de l'aluminium et, éventuellement, d'une fraction de l'élément dopant.

Les alliages de départ mis en oeuvre sont choisis parmi les formes suivantes d'associations binaires nickel/aluminium : NiAl₃, Ni₂Al₃ et proeutectique Al/NiAl₃.

Conformément à l'invention, il est avantageux de choisir le ou les dopants parmi les métaux du groupe IVb des éléments de transition. Le titane s'est avéré comme particulièrement approprié en tant qu'élément d'addition du Ni de Raney. Sur le plan quantitatif, le dopant est préférablement surdosé en alliage précurseur avant attaque alcaline, de façon à tenir compte de son élimination.

En pratique, on préfère un rapport pondéral Ti/Ni variant de 0,6 à 4,5 % pour le catalyseur fini.

Ce procédé s'applique, plus particulièrement mais non limitativement, aux substrats nitriles de formule (I) :

NC―R―CN (I)

dans laquelle R représente un groupement alkylène ou alcénylène, linéaire ou ramifié, ayant de 1 à 12 atomes de carbone, ou un groupement arylène ou aralkylène ou aralcénylène substitué ou non.

De préférence, on met en oeuvre dans le procédé de rinvention des dinitriles de formule (I) dans laquelle R représente un radical alkylène, linéaire ou ramifié ayant de de 2 à 6 atomes de carbone.

A titre d'exemples de tels dinitriles, on peut citer notamment l'adiponitrile, le méthylglutaronitrile, l'éthylsuccinonitrile, le malononitrile, le succinonitrile et le glutaronitrile et leurs mélanges, notamment les mélanges adiponitrile, méthylglutaronitrile, éthylsuccinonitrile qui proviennent d'un même procédé de synthèse de l'adiponitrile.

L'introduction du substrat nitrile, par exemple l'adiponitrile, dans le milieu réactionnel se fait en respectant une concentration comprise entre 0,001 % et 30 % en poids par rapport au poids total (p/p) du milieu réactionnel et de préférence entre 0,1 % et 20 % p/p.

De façon privilégiée, la base forte mise en oeuvre est choisie parmi les composés suivants: LiOH, NaOH, KOH, RbOH, CsOH et leur mélanges.

En pratique, on utilise préférentiellement NaOH et KOH, pour un bon compromis performance-prix, bien que RbOH et CsOH donnent des résultats encore meilleurs.

Le milieu réactionnel d'hydrogénation est de préférence liquide. Il contient au moins un solvant apte à solubiliser le substrat nitrile à hydrogéner, sachant que cette transformation s'opère mieux lorsque ledit substrat se trouve en solution.

Suivant une modalité intéressante du procédé selon l'invention, on utilise un milieu réactionnel liquide au moins partiellement aqueux. L'eau est généralement présente dans une quantité inférieure ou égale à 50 %, avantageusement inférieure ou égale à 20 % en poids par rapport au milieu réactionnel total. Plus préférentiellement encore, la teneur en eau du milieu réactionnel est comprise entre 0,1 et 15 % en poids par rapport à l'ensemble des constituants dudit milieu.

En complément ou en substitution à l'eau, on peut prévoir au moins un autre solvant, du type alcool et/ou amide. Les alcools qui conviennent plus particulièrement sont par exemple le méthanol, l'éthanol, le propanol, l'isopropanol, le butanol, les glycols, tels que l'éthylène et/ou le propylène glycol, des polyols et/ou des mélanges desdits composés.

Dans le cas où le solvant est constitué par un amide, il peut s'agir par exemple du diméthylformamide ou du diméthylacétamide.

Lorsqu'il est employé avec l'eau, le solvant, de préférence alcoolique, représente de deux à quatre parties en poids pour une partie en poids d'eau et de préférence trois parties pour une partie d'eau.

Selon une autre caractéristique préférée de l'invention, on incorpore de l'amine, dont la préparation est visée par le procédé, au sein du milieu réactionnel. Il s'agit par exemple d'hexaméthylènediamine, lorsque le substrat nitrile est l'adiponitrile.

La concentration de l'amine visée dans le milieu réactionnel est avantageusement comprise entre 50 % et 99 % en poids par rapport à la totalité du solvant inclus dans ledit milieu réactionnel et, plus préférentiellement encore, est comprise entre 60 % et 99 % en poids.

La quantité de base dans le milieu réactionnel varie en fonction de la nature du milieu réactionnel.

Dès lors que le milieu réactionnel ne contient que de l'eau et de l'amine visée, à titre de milieu solvant liquide, la quantité de base est avantageusement supérieure ou égale à 0,1 mol/kg de catalyseur, de préférence comprise entre 0,1 et 2 mol/kg de catalyseur et plus préférentiellement encore entre 0,5 et 1,5 mol/kg de catalyseur.

Dans le cas où le milieu réactionnel comprend de l'eau et un alcool et/ou un amide, la quantité de base est supérieure ou égale à 0,05 mol/kg de catalyseur, est comprise de préférence entre 0,1 et 10,0 mol/kg et plus préférentiellement encore entre 1,0 et 8,0 mol/kg.

Une fois arrêtée la composition du milieu réactionnel et le choix du catalyseur, on procède à un mélange de ces deux éléments, puis on chauffe ce mélange à une température réactionnelle inférieure ou égale à 150°C, de préférence inférieure ou égale à 120°C et, plus préférentiellement encore, inférieure ou égale à 100°C.

Concrètement, cette température est comprise entre la température ambiante (20°C environ) et 100°C.

Préalablement, simultanément ou postérieurement au chauffage, l'enceinte réactionnelle est amenée à la pression en hydrogène convenable, c'est-à-dire, en pratique, comprise entre 0,10 et 10 MPa.

La durée de la réaction est variable en fonction des conditions réactionnelles et du catalyseur.

Dans un mode de fonctionnement discontinu, elle peut varier de quelques minutes à plusieurs heures.

Dans un mode de fonctionnement continu, qui est parfaitement envisageable pour le procédé selon l'invention, la durée n'est évidemment pas un paramètre figeable.

Il est à noter que l'homme du métier peut moduler la chronologie des étapes du procédé selon l'invention, selon les conditions opératoires. L'ordre donné ci-avant ne correspond qu'à une forme préférée, mais non limitative, du procédé selon l'invention.

Les autres conditions qui régissent l'hydrogénation (en mode continu ou discontinu) conforme à l'invention, relèvent de dispositions techniques traditionnelles et connues en elles-mêmes.

Grâce à toutes les dispositions avantageuses évoquées ci-dessus, le procédé de l'invention permet d'hydrogéner des substrats nitriles en amines, de façon sélective, rapide, commode et économique.

Ce procédé est parfaitement adapté pour transformer l'adiponitrile en hexaméthylènediamine précurseur de polyamide-6,6 ou en aminocapronitrile précurseur de polyamide-6.

L'invention sera mieux comprise et ses avantages et ses variantes de mise en oeuvre ressortiront bien des exemples qui suivent illustrant, de façon non limitative, le procédé d'hydrogénation selon l'invention, y compris la préparation du catalyseur.

### EXEMPLES

### PROTOCOLE GENERAL DE PREPARATION DU CATALYSEUR D'HYDROGENATION NI DE RANEY DOPE AU TITANE.

### 1. DOPAGE METALLURGIQUE

Différentes phases solides d'alliages précurseurs Ni-Al sont disponibles, à savoir : NiAl₃, Ni₂Al₃, NIAI, Ni₃Al, proeutectique Al/NiAl₃, eutectique Al/NiAl₃ et leurs mélanges.

Dans ces exemples, on teste :
- l'alliage proeutectique avec un rapport pondéral Ni/Al de (28-x)/72, contenant x % en poids par poids de Ti, brut de coulée,
- l'alliage NiAl₃ ayant un rapport pondéral Ni/Al de (42-x)/58 et contenant x % en poids par poids de Ti, recuit 48 h à 835°C,
- l'alliage Ni₂Al₃ ayant un rapport pondéral Ni/Al de (58-x)/42 et contenant x % en poids par poids de Ti, recuit 48 h à 940°C.

Le titane employé provient de baguettes de pureté > 99,9 %.

Différents rapports pondéraux Ti/Ni dans l'alliage de départ sont mis en oeuvre : 1 %, 2 %, 3 % et 4 %.

Chaque alliage est porté à une température de fusion qui lui est propre.

### 2. ATTAQUE ALCALINE

Dans un ballon en Téflon® de 2 l, on introduit 300 ml de soude 6N à température ambiante.

10,00 g des alliages ci-dessus sont, par ailleurs, pesés.

On introduit, à la spatule, l'alliage dans de la soude à la vitesse de 20 g/h et de façon à ce que la température moyenne du milieu ne dépasse pas 50°C.

Lorsque tout l'alliage est additionné, on attend la fin de l'effervescence. Après 2 h de reflux, on élimine la solution sumageante par décantation du solide. On lave le catalyseur avec une solution de soude 1N bouillante, puis on le replace dans une solution de soude 6N bouillante. Après 2 h de reflux, on procède à des lavages du catalyseur par des solutions de soude bouillantes de concentrations décroissantes 6N, 3N, 2N et 1N.

Le solide est récupéré dans un flacon et stocké sur soude 1N froide.

### EXEMPLES 1 A 7 ET ESSAIS COMPARATIFS A ET B

### HYDROGENATION EN MODE DISCONTINU : TEST CATALYTIQUE

### 1. APPAREILLAGE:

Le réacteur est un autoclave de 150 ml en acier inocydable 316 L. Il est équipé d'un système d'agitation magnétique (1 500 tr/min, barreau magnétique et contre-pales) assurant un bon transfert gaz-liquide. Le chauffage se fait au moyen d'un manchon chauffant thermorégulé. Le substrat à hydrogéner est introduit par l'intermédiaire d'une ampoule en acier surmontant l'autoclave. Il peut aussi être introduit à l'aide d'une pompe haute pression dans le cas d'un réacteur semi-continu.L'hydrogène est stocké sous 5 MPa dans une réserve munie d'un manomètre relié à un enregistreur. Il est détendu dans le montage à la pression constante de la réaction. La cinétique de la réaction est suivie par enregistrement de la baisse de pression dans la réserve d'hydrogène. Les échantillons d'hydrogénats, destinés à l'analyse, sont prélevés par l'intermédiaire d'un tube plongeant muni d'un filtre en acier.

### 2. PRODUITS UTILISES:

- Adiponitrile à 99,9 % (RHONE-POULENC, PM = 108,15),
- Hexaméthylènediamine à 99,9 % (RHONE-POULENC), PM = 116,21),
- Hydrogène U à 99,995 % en volume,
- Ethanol à 99,8 %,
- Eau distillée,
- Soude à 98 %, potasse à 86 %,
- Catalyseur : nickel de Raney dopé Ti décrit ci-avant.

### 3. DEROULEMENT D'UN ESSAI TYPE DISCONTINU:

### 3.1. CHARGES:

- Adiponitrile (ADN) : 6,0 g (0,055 mole),
- Hydrogène: excès (> 0,222 mole),
- Milieu réactionnel: hexaméthylènediamine (HMD), H₂O et généralement EtOH formant le solvant réactionnel + base alcaline NaOH ou KOH : 42,0 g, (0,10 % de NaOH ou KOH dans le milieu réactionnel),
- Catalyseur : 0,40 g.

### 3.2. MODE OPERATOIRE

On prélève un excès de bouillie de nickel de Raney (1-2g) et on lave le catalyseur avec six fois 50 ml d'eau distillée. On pèse exactement 0,40 g de catalyseur au pycnomètre. Le nickel de Raney humide est ensuite introduit dans l'autoclave. Pour une masse de 0,40 g de catalyseur, la quantité d'eau couramment entraînée est de l'ordre de 0,4 g. Cette masse d'eau sera prise en compte dans la composition du solvant réactionnel qui devra être de 60/30/10 en HMD/éthanol/eau (exemples 1 à 6 et essais comparatifs A et B) ou 98/2 HMD/eau (exemple 7). La base alcaline est introduite avec la quantité d'eau nécessaire à l'ajustement des pourcentages en eau requis. L'ensemble de ces manipulations doit se dérouler sous un ciel d'argon pour minimiser la carbonatation du solvant et l'oxydation du catalyseur.

L'autoclave est ensuite purgé à l'azote et à l'hydrogène. Le réacteur est alors chauffé à 80°C et maintenu sous 2,5 MPa d'hydrogène. L'enregistrement de lapression dans la réserve d'hydrogène est mis en route et l'ADN est additionné rapidement Quand la consommation d'hydrogène devient nulle, le réacteur est encore laissé sous agitation pendant une demi-heure pour mieux apprécier la fin de la réaction.

En fin d'essai, un échantillon d'hydrogénat est prélevé pour la détermination de la sélectivité. L'activité initiale et une "activité moyenne" sont déduites de la courbe de consommation d'hydrogène en fonction du temps.

### 3.3. MESURE DE L'ACTIVITE

La pente à l'origine de la courbe de consommation d'hydrogène est proportionnelle à la vitesse initiale (Vi) qui a une signification cinétique. Cette grandeur est calculée en faisant le quotient à l'origine du nombre de moles d'hydrogène consommé par unité de temps ramené à l'unité de masse de catalyseur. La vitesse initiale sera exprimée en kmole d'hydrogène consommé par kg de catalyseur et par seconde.

Pour une bonne appréciation des performances d'un catalyseur, il est nécessaire de savoir si l'activité initiale n'est pas obérée par un vieillissement prématuré. C'est pourquoi, on mesure également la vitesse moyenne de réaction (Vm), qui est le quotient du nombre de moles d'hydrogène mises en jeu au temps total de la réaction par unité de masse de catalyseur et par seconde.

La reproductibilité du test pour la détermination de Vi et Vm donne une incertitude inférieure à 10 %.

### 3.4. MESURE DE LA SELECTIVITE (S)

En fin de réaction un échantillon d'hydrogénat est prélevé et dilué environ 40 fois dans l'isopropanol. Cet échantillon est analysé quantitativement par chromatographie en phase gazeuse (CPG) à l'aide d'une colonne semi-capillaire). Le détecteur est à ionisation de flamme. La détermination quantitative des sous-produits de la réaction d'hydrogénation de l'ADN est réalisée par la méthode de l'étalon interne (undécane).

La liste des principaux sous-produits dosés est donnée ci-après :
HMI : Hexaméthylèneimine
AMCPA : Aminométhylcyclopentylamine
AZCHe : Azacycloheptène
NEtHMD : N - éthylhexaméthylènediamine
DCH : Diaminocyclohexane cis et trans
BHT : Bis(hexaméthylènetriamine).

La sélectivité (S) en HMD en pourcentage est donnée par la relation : 100 - somme des sélectivités des sous-produits. En effet l'HMD étant mise en oeuvredans le solvant réactionnel, elle ne peut pas être dosée directement de manière très précise. Par contre il a été vérifié que les sous-produits sont globalement tous identifiés.

Les sélectivités en chacun des sous-produits sont représentées par le pourcentage molaire du sous-produit formé par rapport à l'ADN transformé. Dans tous les exemples et essais comparatifs effectués, le taux de transformation de l'ADN (ainsi que celui de l'aminocapronitrile intermédiaire) est de 100 %.

Le taux d'insaturés présents dans l'hydrogénat peut être évalué par polarographie.

### 3.5. RESULTATS DES EXEMPLES ET ESSAIS COMPARATIFS

### Exemples 1 à 6 et essais comparatifs A et B:

HMD (60)/EtOH (30)/H₂O (10)] + NaOH à raison de 0,10 % en poids par rapport au poids de HMD/EtOH/H₂O.

Les performances catalytiques de Nickels de Raney dopés au chrome (essais comparatifs A et B), ont été évaluées dans les mêmes conditions de pression, de température et de milieu réactionnel que dans les essais 2 et 6.

Le catalyseur de l'essai A est obtenu à partir d'un alliage NiAl₃. Son rapport pondéral Cr/Ni est de 0,6 %.

Le catalyseur de l'essai B est obtenu à partir d'un alliage Ni₂Al₃. Son rapport pondéral Cr/Ni est de 3,5 %.

### Exemple 7 :

HMD (98)/H₂O (2) + KOH à 0,01 % par rapport à HMD/H₂O.

Le tableau 3 ci-après présente les résultats obtenus.

**Tableau 1**

| Essais | Alliage Ni/Al % p/p | Dopant % en pds/Ni | Vi | Vm | S % en HMD | S % en DCH |
|---|---|---|---|---|---|---|
| Ex 1 | 26/72 | 1,4 % Ti | 111 | 50 | 97,2 | 0,024 |
| Ex 2 | 40/58 | 1,2 % T | 89 | 46 | 96,8 | 0,028 |
| Ex 3 | 57/42 | 0,73 % T | 49 | 20 | 96,4 | 0,054 |
| Ex 4 | 56/42 | 1,33 % T | 37 | 12 | 96,6 | 0,036 |
| Ex 5 | 55/42 | 2,48 % Ti | 44 | 17 | 95,8 | 0,039 |
| Ex 6 | 54/42 | 3,48 % Ti | 37 | 15 | 96,2 | 0,039 |
| Ec A | 43/58 | 0,6 % Cr | 37 | 16 | 97,1 | 0,044 |
| Ec B | 54/42 | 3,5 % Cr | 145 | 55 | 96,8 | 0,065 |
| Ex 7 | 55/42 | 2,48 % Ti | 21 | 4 | 95,6 | 0,037 |

On note que les impuretés DCH (cis + trans) sont présentes en très faibles quantités. Cela constitue un avantage conséquent à mettre au crédit de l'invention, car ces impuretés ont quasiment la même température d'ébullition que l'HMD et sont donc très difficiles à éliminer.

Pour des sélectivités comparables en HMD, les catalyseurs dopés au Ti permettent de limiter sensiblement la teneur en impuretés DCH : 0,028 % (exemple 2) contre 0,044 % (essai comparatif A). et 0,039 % (exemple 6) contre 0,065 % (essai comparatif B).

## Revendications

1. Procédé d'hydrogénation de nitriles en amines, à l'aide d'un catalyseur de type nickel de Raney, ledit catalyseur étant dopé par au moins un élément métallique d'addition choisi dans le groupe IVb de la classification périodique des éléments et étant issu d'un alliage précurseur métallurgique Ni/Al/élément(s) dopant(s), caractérisé en ce que le procédé consiste essentiellement :
- à sélectionner un milieu réactionnel liquide et solvant du substrat nitrile à hydrogéner,
- à mettre en oeuvre au moins une base minérale choisie parmi les hydroxydes de métal alcalin ou alcalinoterreux,
- à adopter un catalyseur dont le rapport pondéral élément dopant/Ni est compris entre 0,05 et 10 %,
- et à opérer à une température réactionnelle inférieure ou égale à 150°C et sous une pression en hydrogène comprise entre 0,10 MPa et 10 MPa.

2. Procédé selon la revendication 1, caractérisé en ce que l'on met en oeuvre un substrat nitrile de formule (I) :
NC―R―CN (I)
dans laquelle R représente un groupement alkylène ou alcénylène, linéaire ou ramifié, ayant de 1 à 12 atomes de carbone, ou un groupement arylène ou aralkylène ou aralcénylène substitué ou non,
et de préférence un nitrile de formule (I) dans laquelle R représente un radical alkylène, linéaire ou ramifié ayant de de 2 à 6 atomes de carbone.

3. Procédé selon la revendication 2, caractérisé en ce que le substrat nitrile est choisi parmi l'adiponitrile, le méthylglutaronitrile, l'éthylsuccinonitrile, le malononitrile, le succinonitrile et le glutaronitrile et leurs mélanges.

4. Procédé selon l'une quelconque des revendications 1 à 3, caractérisé en ce que le substrat, caractérisé en ce que l'on fixe la concentration en substrat nitrile dans le milieu réactionnel total à une valeur comprise entre 0,001 % et 30 % en poids par poids et de préférence entre 0,1 % et 20 %.

5. Procédé selon l'une quelconque des revendications 1 à 4, caractérisé en ce que la base mise en oeuvre est constituée par au moins l'un des composés suivants : LiOH, NaOH, KOH, RbOH, CsOH.

6. Procédé selon l'une quelconque des revendications 1 à 5, caractérisé en ce que le milieu réactionnel liquide comprend de l'eau, de préférence dans une quantité inférieure ou égale à 20 % en poids du milieu réactionnel liquide total et, plus préférentiellement encore, comprise entre 0,1 % et 15 % en poids.

7. Procédé selon l'une quelconque des revendications 1 à 6, caractérisé en ce que le milieu réactionnel liquide contient de l'amine visée.

8. Procédé selon la revendication 7, caractérisé en ce que l'amine visée est introduite dans le milieu réactionnel liquide, à raison de 50 à 99 % et préférentiellement à raison de 60 à 99 % en poids par rapport au poids du milieu réactionnel liquide total.

9. Procédé selon l'une des revendications 1 à 8, caractérisé en ce que l'on met en oeuvre un milieu réactionnel liquide comprenant un alcool et/ou un amide.

10. Procédé selon la revendication 9, caractérisé en ce que l'alcool est sélectionné parmi les composés suivants : méthanol, éthanol, propanol, isopropanol, butanol, glycols tels que éthylèneglycol et/ou propylène glycol, polyols et leurs mélanges.

11. Procédé selon la revendication 9, caractérisé en ce que l'amide est le diméthylformamide et/ou le diméthylacétamide.

12. Procédé selon l'une quelconque des revendications 1 à 8, caractérisé en ce que l'on utilise la base dans une quantité supérieure ou égale à 0,1 mol/kg de catalyseur, de préférence comprise entre 0,1 et 2,0 mol/kg de catalyseur et, plus préférentiellement encore, entre 0,5 et 1,5 mol/kg de catalyseur.

13. Procédé selon l'une quelconque des revendications 9 à 11, caractérisé en ce que l'on utilise la base dans une quantité supérieure ou égale à 0,05 mol/kg de catalyseur, de préférence comprise entre 0,1 et 10,0 mol/kg et, plus préférentiellement encore, entre 1,0 et 8,0 mol/kg.

14. Procédé selon l'une quelconque des revendications 1 à 13, caractérisé en ce que l'on effectue l'hydrogénation à une température de milieu réactionnel de préférence inférieure ou égale à 120°C et, plus préférentiellement encore, inférieure ou égale à 100°C.

## Patentansprüche

1. Verfahren zur Hydrierung von Nitrilen zu Aminen mit Hilfe eines Katalysators vom Typ Raney-Nickel, wobei besagter Katalysator mit wenigstens einem metallischen Legierungselement dotiert ist, das aus der Gruppe IVb des Periodensystems der Elemente ausgewählt ist, und aus einer metallurgischen Vorläuferlegierung Ni/Al/Dotierungselement(e) hervorgegangen ist, dadurch gekennzeichnet, daß das Verfahren im wesentlichen besteht aus:
- dem Auswählen eines flüssigen Reaktionsmediums und Lösungsmittels für das zu hydrierende Nitrilsubstrat,
- der Verwendung wenigstens einer Mineralbase, die unter den Alkali- oder Erdalkalimetallhydroxiden ausgewählt ist,
- der Wahl eines Katalysators, dessen Gewichtsverhältnis Dotierungselement/Ni zwischen 0,05 und 10% liegt,
- und dem Arbeiten bei einer Reaktionstemperatur, die niedriger oder gleich 150°C ist, und unter einem Wasserstcffdruck zwischen 0,10 MPa und 10 MPa.

2. Verfahren gemäß Anspruch 1, dadurch gekennzeichnet, daß man ein Nitrilsubstrat der Formel (I):
NC―R―CN (I)
in der R eine lineare oder verzweigte Alkylen- oder Alkenylengruppe mit 1 bis 12 Kohlenstoffatomen oder eine substituierte oder nicht substituierte Arylen- oder Aralkylen- oder Aralkenylengruppe darstellt,
und vorzugsweise ein Nitril der Formel (I), in der R einen linearen oder verzweigten Alkylenrest mit 2 bis 6 Kohlenstoffatomen darstellt, verwendet.

3. Verfahren gemäß Anspruch 2, dadurch gekennzeichnet, daß das Nitrilsubstrat unter Adiponitril, Methylglutaronitril, Ethylsuccinonitril, Malononitril, Succinonitril und Glutaronitril und deren Gemischen ausgewählt ist.

4. Verfahren gemäß einem der Ansprüche 1 bis 3, dadurch gekennzeichnet, daß man die Konzentration an Nitrilsubstrat in dem gesamten Reaktionsmedium auf einen Wert zwischen 0,001 Gew.-% und 30 Gew.-% und vorzugsweise zwischen 0,1 und 20 Gew.-% festlegt.

5. Verfahren gemäß einem der Ansprüche 1 bis 4, dadurch gekennzeichnet, daß die verwendete Base aus wenigstens einer der folgenden Verbindungen besteht: LiOH, NaOH, KOH, RbOH, CsOH.

6. Verfahren gemäß einem der Ansprüche 1 bis 5, dadurch gekennzeichnet, daß das flüssige Reaktionsmedium Wasser vorzugsweise in einer Menge umfaßt, die kleiner oder gleich 20% des Gewichts des gesamten flüssigen Reaktionsmediums ist und noch stärker bevorzugt zwischen 0,1% und 15 Gew.-% liegt.

7. Verfahren gemäß einem der Ansprüche 1 bis 6, dadurch gekennzeichnet, daß das flüssige Reaktionsmedium das angestrebte Amin enthält.

8. Verfahren gemäß Anspruch 7, dadurch gekennzeichnet, daß das angestrebte Amin in einer Menge von 50 bis 99 Gew.-% und vorzugsweise von 60 bis 99 Gew.-%, bezogen auf das Gewicht des gesamten flüssigen Reaktionsmediums, in das flüssige Reaktionsmedium eingeführt wird.

9. Verfahren gemäß einem der Ansprüche 1 bis 8, dadurch gekennzeichnet, daß man ein flüssiges Reaktionsmedium, das einen Alkohol und/oder ein Amid umfaßt, verwendet.

10. Verfahren gemäß Anspruch 9, dadurch gekennzeichnet, daß der Alkohol unter den folgenden Verbindungen ausgewählt ist: Methanol, Ethanol, Propanol, Isopropanol, Butanol, Glykole wie Ethylenglykol und/oder Propylenglykol, Polyole und deren Gemische.

11. Verfahren gemäß Anspruch 9, dadurch gekennzeichnet, daß das Amid Dimethylformamid und/oder Dimethylacetamid ist.

12. Verfahren gemäß einem der Ansprüche 1 bis 8, dadurch gekennzeichnet, daß man die Base in einer Menge verwendet, die größer oder gleich 0,1 mol/kg Katalysator ist, vorzugsweise zwischen 0,1 und 2,0 mol/kg Katalysator und noch stärker bevorzugt zwischen 0,5 und 1,5 mol/kg Katalysator liegt.

13. Verfahren gemäß einem der Ansprüche 9 bis 11, dadurch gekennzeichnet, daß man die Base in einer Menge verwendet, die größer oder gleich 0,05 mol/kg Katalysator ist, vorzugsweise zwischen 0,1 und 10,0 mol/kg und noch stärker bevorzugt zwischen 1,0 und 8,0 mol/kg liegt.

14. Verfahren gemäß einem der Ansprüche 1 bis 13, dadurch gekennzeichnet, daß man die Hydrierung bei einer Temperatur des Reaktionsmediums ausführt, die vorzugsweise niedriger oder gleich 120 °C und noch stärker bevorzugt niedriger oder gleich 100 °C ist.

## Claims

1. Process for hydrogenation of nitriles to amines using a catalyst of Raney nickel type,
characterized in that:
- the said catalyst is doped with at least one additional metal element chosen from group IVb of the periodic classification of the elements and results from an Ni/Al/doping element(s) metallurgic precursor alloy,
and in that the process consists essentially:
- in selecting a liquid reaction medium which dissolves the nitrile substrate to be hydrogenated,
- in using at least one inorganic base chosen from alkali metal or alkaline-earth metal hydroxides,
- in adopting a catalyst whose doping element/Ni ratio by weight is between 0.05 and 10%,
- and in carrying out the reaction at a temperature which is less than or equal to 150°C and a hydrogen pressure of between 0.10 MPa and 10 MPa.

2. Process according to Claim 1,
characterized in that use is made of a nitrile substrate of formula (I) :
NC-R-CN (I)
in which R represents a linear or branched alkylene or alkenylene group having from 1 to 12 carbon atoms or a substituted or unsubstituted arylene or aralkylene or aralkenylene group,
and preferably a nitrile of formula (I) in which R represents a linear or branched alkylene radical having from 2 to 6 carbon atoms.

3. Process according to Claim 2, characterized in that the nitrile substrate is chosen from adiponitrile, methylglutaronitrile, ethylsuccinonitrile, malononitrile, succinonitrile and glutaronitrile and their mixtures.

4. Process according to any one of Claims 1 to 3, characterized in that the concentration of nitrile substrate in the total reaction medium is set at a value of between 0.001% and 30% weight for weight and preferably between 0.1% and 20%.

5. Process according to any one of Claims 1 to 4, characterized in that the base used consists of at least one of the following compounds: LiOH, NaOH, KOH, RbOH or CsOH.

6. Process according to any one of Claims 1 to 5, characterized in that the liquid reaction medium comprises water, preferably in an amount less than or equal to 20% by weight of the total liquid reaction medium and, more preferentially still, between 0.1% and 15% by weight.

7. Process according to any one of Claims 1 to 6, characterized in that the liquid reaction medium contains targeted amine.

8. Process according to Claim 7, characterized in that the targeted amine is introduced into the liquid reaction medium in a proportion of 50 to 99% and preferentially in a porportion of 60 to 99% by weight with respect to the weight of the total liquid reaction medium.

9. Process according to one of Claims 1 to 8, characterized in that use is made of a liquid reaction medium comprising an alcohol and/or an amide.

10. Process according to Claim 9, characterized in that the alcohol is selected from the following compounds: methanol, ethanol, propanol, isopropanol, butanol, glycols, such as ethylene glycol and/or propylene glycol, polyols and their mixtures.

11. Process according to Claim 9, characterized in that the amide is dimethylformamide and/or dimethylacetamide.

12. Process according to any one of Claims 1 to 8, characterized in that the base is used in an amount greater than or equal to 0.1 mol/kg of catalyst, preferably between 0.1 and 2.0 mol/kg of catalyst and, more preferentially still, between 0.5 and 1.5 mol/kg of catalyst.

13. Process according to any one of Claims 9 to 11, characterized in that the base is used in an amount greater than or equal to 0.05 mol/kg of catalyst, preferably between 0.1 and 10.0 mol/kg and, more preferentially still, between 1.0 and 8.0 mol/kg.

14. Process according to any one of Claims 1 to 13, characterized in that the hydrogenation is carried out at a temperature of the reaction medium which is preferably less than or equal to 120°C and, more preferentially still, less than or equal to 100°C.
